# EUROPEAN PATENT APPLICATION

(11) **EP 2 123 268 A1**
(43) Date of publication of application: **25.11.2009**
(21) Application number: 08711620.8
(22) Date of filing: 20.02.2008
(51) Int. Cl.: A61K 31/165, A61K 31/4453, A61K 31/5375, A61P 25/02, A61P 27/02, A61P 27/04, A61P 27/14

(54) **PHARMACEUTICAL COMPOSITION FOR PREVENTION OR TREATMENT OF DISEASE ASSOCIATED WITH TEAR REDUCTION**

(30) Priority: 21.02.2007 JP 2007040191
(71) Applicant: Kissei Pharmaceutical Co., Ltd., Matsumoto-shi Nagano 399-8710 (JP)
(72) Inventor: KOBAYASHI, Mamoru, Azumino-shi Nagano 399-8304 (JP); ASARI, Tetsuya, Azumino-shi Nagano 399-8304 (JP); TADACHI, Mariko, Azumino-shi Nagano 399-8304 (JP)
(74) Representative: Behnisch, Werner
(86) International application number: PCT/JP2008/052814
(87) International publication number: WO 2008/102790

(57) **Abstract**

The present invention provides pharmaceutical compositions for the prevention or treatment of diseases associated with decrease in tear.

The present invention provides pharmaceutical compositions for the prevention or treatment of diseases associated with decrease in tear such as dry eye, dry disorders of cornea and conjunctiva, disorders of the keratoconjunctival epithelium, syndrome with decrease in tear secretion, xerophthalmia, dry eye due to aging, ophthalmopathy in Stevens-Johnson syndrome, ophthalmopathy in Sjögren's syndrome, keratoconjunctival ulcer, dryness in wearing of contact lens or the like, which comprises as an active ingredient a phenylethanolaminotetralincarboxamide derivative represented by the general formula (I) wherein A represents a lower alkylene group, B represents an amino group, a di(lower alkyl)amino group or a 3 to 7-membered alicyclic amino group which may have an oxygen atom in the ring, a carbon atom with the mark "*" represents a carbon atom of S-configuration or R-configuration, or a mixture thereof and a carbon atom with (S) represents a carbon atom of S-configuration, or a pharmaceutically acceptable salt thereof.

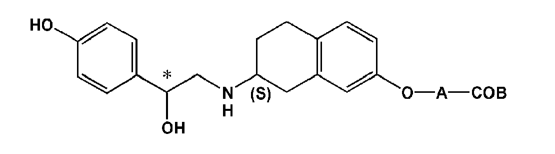

## Description

### Field of the invention

The present invention relates to a pharmaceutical composition useful for the prevention or treatment of a disease associated with decrease in tear.

More specifically, the present invention relates to a pharmaceutical composition comprising as an active ingredient a phenylethanolaminotetralincarboxamide derivative represented by the general formula: 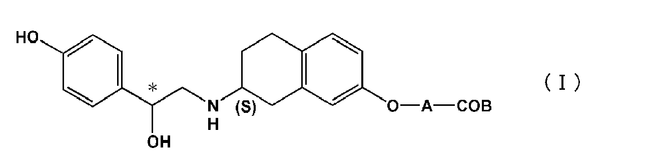 wherein A represents a lower alkylene group, B represents an amino group, a di(lower alkyl)amino group or a 3 to 7-membered alicyclic amino group which may have an oxygen atom in the ring, a carbon atom with the mark "*" represents a carbon atom of S-configuration or R-configuration or a mixture thereof, and a carbon atom with (S) represents a carbon atom of S-configuration, or a pharmaceutically acceptable salt thereof, AJ-9766, FR-149175 or N-5984 or the like, which is useful for the prevention or treatment of diseases associated with decrease in tear and the like.

### Background Art

A typical disease associated with decrease in tear is dry eye. By the Dry Eye Study Group, dry eye is defined as disorders of the keratoconjunctival epithelium caused by qualitative or quantitative abnormality of tear (lacrimal layer) and diagnostic criteria of dry eye based on examination of qualitative and quantitative abnormalities of the tear (ocular layer) and disorders of the keratoconjunctival epithelium have been proposed (for example, see Non-patent reference 1). The diagnostic criteria have been reinvestigated every 10 years, and the 1995 criteria are currently used widely in Japan.
Overseas, the NIH diagnostic criteria (Lemp et al. 1995) are used in general. As major subjective symptoms of dry eye, dryness, pain, itching of eyes, blurring of vision due to disorders of the keratoconjunctival epithelium, severe vision disorders and the like are known.

In addition to dry eye, the following diseases can be considered to be syndromes with similar symptoms that are associated with decrease in tear: dry disorders of cornea and conjunctiva, disorders of the keratoconjunctival epithelium, syndrome with decrease in tear secretion, xerophthalmia, dry eye due to aging, ophthalmopathy in Stevens-Johnson syndrome, ophthalmopathy in Sjögren's syndrome, keratoconjunctival ulcer, oligodacrya, keratoconjunctivitis sicca, ocular pemphigus, blepharitis marginalis, insufficient occlusion of eye lids, sensory neuroparalysis, allergic conjunctivitis, and dryness post-viral conjunctivitis, post-cataract surgery, in wearing of contact lens or in operation of visual display terminal (VDT).

The principal treatment of these diseases associated with decrease in tear is pharmacotherapy using ophthalmic preparations, although surgeries such as plug in the lacrimal puncta and punctual occlusion are also performed. Principally employed pharmacotherapy includes artificial tears for the purpose of increasing tear and eye drops of sodium hyaluronate for the purpose of stabilizing the tear on the keratoconjunctival epithelium. However, whereas the aqueous layer, lipid layer and mucous layer, which form the lacrimal layer, are said to be important to maintain the healthy keratoconjunctival surface, drugs used currently are not sufficiently effective. When it is caused by allergy or inflammation, steroidal and anti-allergic eye drops are used. However, adverse reactions such as increase in intraocular pressure induced by prolonged administration of steroids are sometimes problematic. The number of patients continues to increase, as the environment of patients changes like increase in operations with staring at OA instruments, an air pollution and allergy. Thus, early development of an effective therapeutic agent is desired (for example, see Non-patent reference 2).

There are two factors in decrease in tear. One is decrease in tear secretion and the other is increase in evaporation and excretion of tear. Tear secretion mainly occurs in two ways. One is reflex secretion induced by stimulation to the region controlled by the trigeminal nerve (cornea, conjunctiva, skin, nose, emotion and the like.). The other is basic secretion, which protects the keratoconjunctival surface. In nerve pathways that facilitate tear secretion, there are three of the trigeminal, parasympathetic and sympathetic nerves (see Non-patent reference 3). Although reflex secretion is said to be provided chiefly by the main lacrimal gland, other secretory glands may be playing roles (see Non-patent reference 4). It is reported that the main lacrimal gland is controlled by the sympathetic and parasympathetic nerves or neuropeptides such as neuropeptide Y. As tear secretion is suppressed by β-adrenoceptor blockers such as propranolol, it is thought that β₁ adrenoceptor (hereinafter referred to as "β₁ AR") or β₂ adrenoceptor (hereinafter referred to as "β₂ AR") subtype plays a role in tear secretion (see Non-patent reference 5). On the other hand, lipid secretion, which prevents the evaporation of tear, originates in the meibomian glands, Zeis glands and Moll glands (see Non-patent reference 4), and is controlled by the sympathetic and parasympathetic nerves and neuropeptides such as substance P. In addition, it has been elucidated that glycoproteins such as mucin that forms the mucous layer are secreted from goblet cells and mucous cells in the lacrimal gland (see Non-patent reference 6). However, the detailed mechanism of the secretion remains unclear. It is said that hypofunction of these lipid and mucous secretion causes the breakdown of the lacrimal three-layer structure of the keratoconjunctiva and plays an important role in dry eye as a result.

Tear supplied by the lacrimal gland and keratoconjunctiva contains bioactive substances such as various electrolytes, proteins and vitamin A. Among proteins secreted in tear, mucin is known as a glycoprotein originating from the keratoconjunctiva.
Mucin is useful for the retention of wettability of the keratoconjunctiva surface, and protect the keratoconjunctiva as a lubricant against eyeblink movement (see Non-patent reference 3). In addition, as proteins originating from the lacrimal gland, mucin, lactoferrin, lipocaine, IgA, complement, fibronectin, EGF (epithelial growth factor), HGF (hepatocellular growth factor), TGF (transforming growth factor)β₁, TGFβ₂, various cytokines, amylase, SOD (superoxide dismutase), lysozyme and the like are known. These proteins are thought to be in charge of prevention of evaporation of moisture, antibacterial action and nutrition supply to the ophthalmic tissues and the like. Development of a drug that repairs injured sites of the keratoconjunctival epithelium in dry eye and other diseases and prevents the exacerbation by facilitating the secretion of tear which contains these proteins are desired. It is expected that a substance having a facilitating effect of mucin secretion in tear is useful for the treatment of corneal disorders such as keratitis wherein disorders in the corneal epithelium are observed, conjunctivitis, corneal epithelial abrasion, cornel ulcer and the like as well as dry eye.

It has been known that the phenylethanolaminotetralincarboxamide derivatives represented by the above general formula (I) have a β₂ AR stimulating activity and are useful for the prevention of threatened abortion and premature labor, prevention or treatment of diseases associated with bronchiostenosis and airway obstruction, and pain remission and promoting stone removal in urolithiasis (see Patent reference 1). In addition, it has been reported that one of the present phenylethanolaminotetralincarboxamide derivatives, 2-[(2S)-2-[[(2R)-2-hydroxy-2-(4-hydroxyphenyl)ethyl]amino]-1,2,3,4-tetrahydronaphthalen-7-yloxy]-N,N-dimethylacetamide, has both β₂ AR stimulating activity and β₃ adrenoceptor (hereinafter referred to as "β₃ AR") stimulating activity (see Non-patent reference 7). However, it is not described nor suggested that the above phenylethanolaminotetralincarboxamide derivatives represented by the above general formula (I) increase the quantities of tear and protein in tear, or are useful for diseases associated with decrease in tear.

It has been known that AJ-9677 (chemical name: 3-[(2R)-[[(2R)-(3-chlorophenyl)-2-hydroxyethyl]amino]propyl-1H-indol-7-yloxy]acetic acid, see Non-patent reference 8), FR-149175 (chemical name: ethyl [(S)-8-[(R)-2-(3-chlorophenyl)-2-hydroxyethylamino]-6,7,8,9-tetrahydro-5H-benzocyclohepten-2-yloxy]acetate monohydrochloride monohydrate, see Non-patent reference 9) and N-5984 (chemical name: 6-[2-(R)-[[2-(R)-(3-chlorophenyl)-2-hydroxyethyl]amino]propyl]-2,3-dihydro-1,4-benzodioxine-2-(R)-carboxylic acid, see Non-patent reference 10, hereinafter referred to as "KRP-204") have a β₃ AR stimulating activity. However, it is not known that these compounds increase the quantities of tear and protein in tear, or are useful for diseases associated with decrease in tear.

It has been reported that a few β₂ AR stimulants which are β₂-selective compared to their β₁ AR stimulating activity have facilitating effects of tear secretion and/or protein secretion in tear, are useful for the prevention and treatment of dry ophthalmopathy or the like and have an advantage in that they have a less cardioactivity than a β adrenoceptor stimulant having a β₁ AR stimulating activity (Patent reference 2). Nothing, however, has been described about effects facilitating the secretion of mucin, which plays an important role to retain the wettability of the cornea and conjunctiva in the treatment of corneal disorders such as keratitis wherein disorders in the corneal epithelium are observed, conjunctivitis, corneal epithelial abrasion, cornel ulcer and the like as well as dry eye, and any of them has not been practically used.
[Patent reference 1] International publication No. W097-38970 pamphlet
[Patent reference 2] International publication No. WO01-41806 pamphlet
[Non-patent reference 1] Jun Shimazaki et al., Ganka (Ophthalmology), 1995, Vol.37, pp.765-770
[Non-patent reference 2] Edited by Kazuo Tsubota, Dry eye clinic, Igakusyoin, 2000, pp.43-53
[Non-patent reference 3] Edited by Yoshihisa Oguchi et al., Ocular Surface no Shindan to Chiryo (Diagnosis and Treatment of Ocular Surface), Medical Aoi Publication, 1993, pp.15-30
[Non-patent reference 4] Lemp M.A. et al., The lacrimal apparatus. Adler's physiology of the eye, Edit.9, Mosby Year Book company, 1992, pp.18-28
[Non-patent reference 5] Petounis A.D. et al., Int. Ophthalm., 1989, Vol.13, pp.75-80
[Non-patent reference 6] Sullivan D.A. et al., Lacrimal grand, tear film and dry eye syndromes, Plenum Press company, 1994, pp.1-9
[Non-patent reference 7] Masuo Akahane, et al., Naunyn-Schmiedeberg's Arch. Pharmacol., 1998, Vol.358, Suppl.1, p.R518
[Non-patent reference 8] Hiroshi Harada, et al., Chem. Pharm. Bull., 2005, Vol.53, pp.184-198
[Non-patent reference 9] Yoshifumi Hatakeyama, et al., Am J Physiol Regulatory Integrative Comp Physiol, 2004, Vol.287, pp.R336-341
[Non-patent reference 10] Teruyuki Yanagiwsawa, et al., Tohoku J. Exp. Med., 2000, Vol.192, pp.181-193

### Disclosure of the invention

### Problem that the invention aims to solve

The purpose of the present invention is to provide a pharmaceutical composition for the prevention or treatment of a disease associated with decrease in tear.

### Means to solve the problem

As the result of earnest research on the above-mentioned problem, the present inventors newly found that the compounds represented by the above general formula (I), pharmaceutically acceptable salts thereof and the like increase the quantities of tear secretion and protein secretion in tear, and especially, extremely increase the quantities of protein secretion in tear and mucin secretion compared with a selective β2 AR stimulant, and thereby forming the basis of the present invention.

That is, the present invention relates to:
[1] a pharmaceutical composition for the prevention or treatment of a disease associated with decrease in tear, which comprises as an active ingredient a compound selected from a group consisting of a phenylethanolaminotetralincarboxamide derivative represented by the general formula: 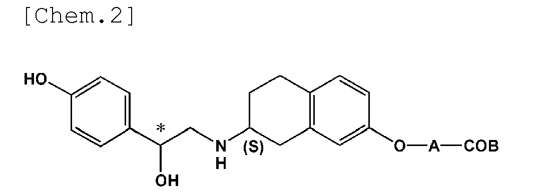 wherein A represents a lower alkylene group, B represents an amino group, a di(lower alkyl)amino group or a 3 to 7-membered alicyclic amino group which may have an oxygen atom in the ring, a carbon atom with the mark "*" represents a carbon atom of S-configuration or R-configuration, or a mixture thereof, and a carbon atom with (S) represents a carbon atom of S-configuration, [3-[(2R)-[[(2R)-(3-chlorophenyl)-2-hydroxyethyl]amino]propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]propyl]-2,3-dihydro-1,4-benzodixine-2-(R)-carboxylic acid, and a pharmaceutically acceptable salt thereof;
[2] a pharmaceutical composition as described in the above [1] wherein the active ingredient is a phenylethanolaminotetralincarboxamide derivative represented by the general formula: 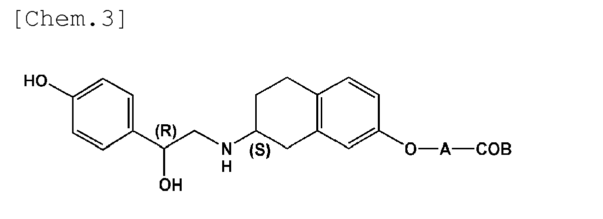 wherein A represents a lower alkylene group, B represents an amino group, a di(lower alkyl)amino group or a 3 to 7-membered alicyclic amino group which may have an oxygen atom in the ring, a carbon atom with (R) represents a carbon atom of R-configuration and a carbon atom with (S) represents a carbon of S-configuration, or a pharmaceutically acceptable salt thereof;
[3] a pharmaceutical composition as described in the above [2] wherein the active ingredient is a compound represented by one of the formulas: 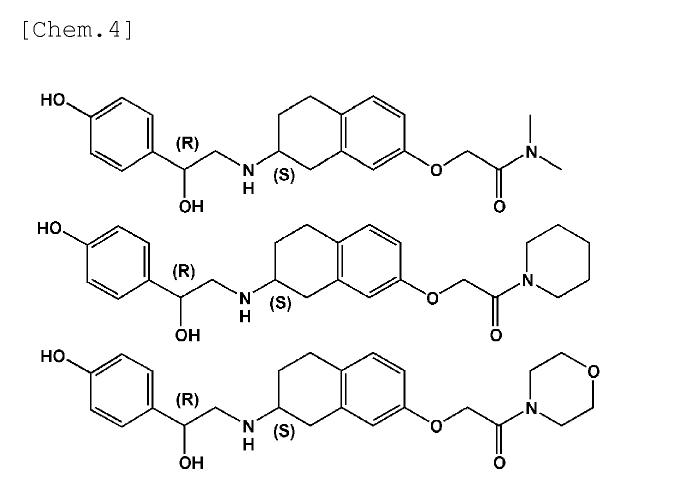 or a pharmaceutically acceptable salt thereof;
[4] a pharmaceutical composition for the enhancement of protein in tear, which comprises as an active ingredient a compound selected from a group consisting of a phenylethanolaminotetralincarboxamide derivative represented by the general formula: 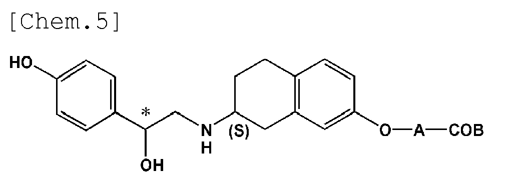 wherein A represents a lower alkylene group, B represents an amino group, a di(lower alkyl)amino group or a 3 to 7-membered alicyclic amino group which may have an oxygen atom in the ring, a carbon atom with the mark "*" represents a carbon atom of S-configuration or R-configuration, or a mixture thereof, and a carbon atom with (S) represents a carbon atom of S-configuration, [3-[(2R)-[[(2R)-(3-chlorophenyl)-2-hydroxyethyl]amino]propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]propyl]-2,3-dihydro-1,4-benzodixine-2-(R)-carboxylic acid, and a pharmaceutically acceptable salt thereof;
[5] a pharmaceutical composition as described in the above [4] wherein the active ingredient is a phenylethanolaminotetralincarboxamide derivative represented by the general formula: 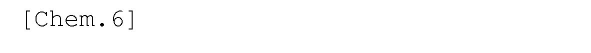 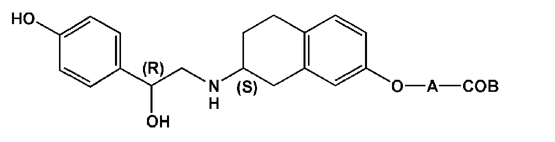 wherein A represents a lower alkylene group, B represents an amino group, a di(lower alkyl)amino group or a 3 to 7-membered alicyclic amino group which may have an oxygen atom in the ring, a carbon atom with (R) represents a carbon atom of R-configuration and a carbon atom with (S) represents a carbon of S-configuration, or a pharmaceutically acceptable salt thereof;
[6] a pharmaceutical composition as described in the above [5] wherein the active ingredient is a compound represented by one of the formulas: 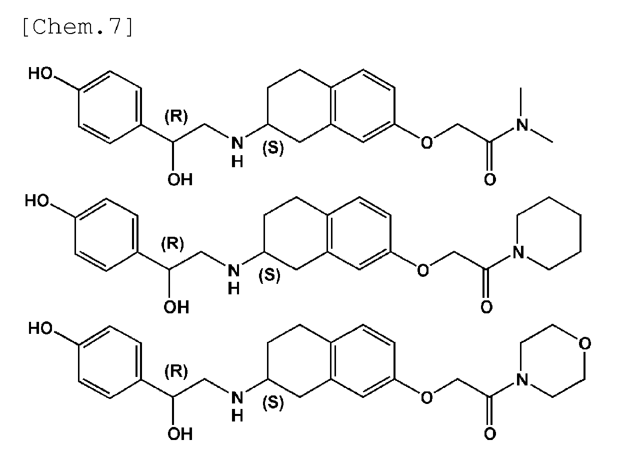 or a pharmaceutically acceptable salt thereof;
[7] a pharmaceutical composition for the facilitation of mucin secretion in tear, which comprises as an active ingredient a compound selected from a group consisting of a phenylethanolaminotetralincarboxamide derivative represented by the general formula: 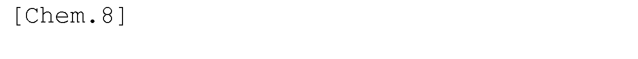 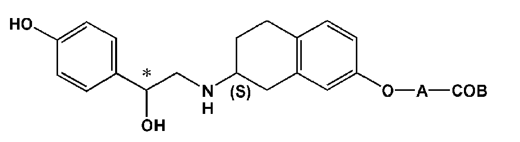 wherein A represents a lower alkylene group, B represents an amino group, a di(lower alkyl)amino group or a 3 to 7-membered alicyclic amino group which may have an oxygen atom in the ring, a carbon atom with the mark "*" represents a carbon atom of S-configuration or R-configuration, or a mixture thereof, and a carbon atom with (S) represents a carbon atom of S-configuration, [3-[(2R)-[[(2R)-(3-chlorophenyl)-2-hydroxyethyl]amino]propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]propyl]-2,3-dihydro-1,4-benzodixine-2-(R)-carboxylic acid, and a pharmaceutically acceptable salt thereof;
[8] a pharmaceutical composition as described in the above [7] wherein the active ingredient is a phenylethanolaminotetralincarboxamide derivative represented by the general formula: 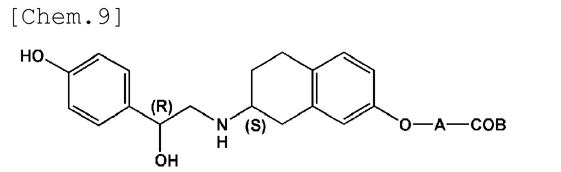 wherein A represents a lower alkylene group, B represents an amino group, a di(lower alkyl)amino group or a 3 to 7-membered alicyclic amino group which may have an oxygen atom in the ring, a carbon atom with (R) represents a carbon atom of R-configuration and a carbon atom with (S) represents a carbon of S-configuration, or a pharmaceutically acceptable salt thereof;
[9] a pharmaceutical composition as described in the above [8] wherein the active ingredient is a compound represented by one of the formulas: 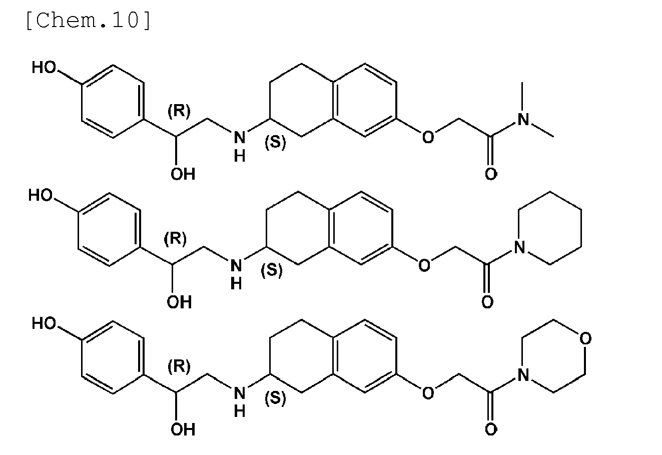 or a pharmaceutically acceptable salt thereof;
[10] a pharmaceutical composition as described in any of the above [1] to [3] 3 wherein the disease associated with decrease in tear are one or more diseases selected from the group consisting of dry eye, dry disorders of cornea and conjunctiva, disorders of the keratoconjunctival epithelium, syndrome with decrease in tear secretion, xerophthalmia, dry eye due to aging, ophthalmopathy in Stevens-Johnson syndrome, ophthalmopathy in Sjögren's syndrome, keratoconjunctival ulcer, oligodacrya, keratoconjunctivitis sicca, ocular pemphigus, blepharitis marginalis, insufficient occlusion of eye lids, sensory neuroparalysis, allergic conjunctivitis, and dryness post-viral conjunctivitis, post-cataract surgery, in wearing of contact lens or in operation of visual display terminal (VDT);
[11] a pharmaceutical composition as described in any of the above [1] to [10] wherein the dosage form is an oral formulation;
[12] a pharmaceutical composition as described in any of the above [1] to [10] wherein the dosage form is a parenteral formulation;
[13] a pharmaceutical composition as described in the above [12] wherein the parenteral formulation is an eye drop;
[14] a method for the prevention or treatment of a disease associated with decrease in tear, which comprises administering an effective amount of a compound selected from the group consisting of a phenylethanolaminotetralincarboxamide derivative as described in any of the above [1] to [3], [3-[(2R)-[[(2R)-(3-chlorophenyl)-2-hydroxyethyl]amino]propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]-propyl]-2,3-dihydro-1,4-benzodixine-2-(R)-carboxylic acid, and a pharmaceutically acceptable salt thereof;
[15] a method for the enhancement of protein in tear, which comprises administering an effective amount of a compound selected from the group consisting of a phenylethanolaminotetralincarboxamide derivative as described in any of the above [4] to [6], [3-[(2R)-[[(2R)-(3-chlorophenyl)-2-hydroxyethyl]amino]propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]propyl]-2,3-dihydro-1,4-benzodixine-2-(R)-carboxylic acid, and a pharmaceutically acceptable salt thereof;
[16] a method for the facilitation of mucin secretion in tear, which comprises administering an effective amount of a compound selected from the group consisting of a phenylethanolaminotetralincarboxamide derivative as described in any of the above [7] to [9], [3-[(2R)-[[(2R)-(3-chlorophenyl)-2-hydroxyethyl]-amino]propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]propyl]-2,3-dihydro-1,4-benzodixine-2-(R)-carboxylic acid, and a pharmaceutically acceptable salt thereof;
[17] a use of a compound selected from the group consisting of a phenylethanolaminotetralincarboxamide derivative as described in any of the above [1] to [3], [3-[(2R)-[[(2R)-(3-chlorophenyl)-2-hydroxyethyl]-amino]propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]propyl]-2,3-dihydro-1,4-benzodixine-2-(R)-carboxylic acid, and a pharmaceutically acceptable salt thereof for manufacturing of a pharmaceutical composition for the prevention or treatment of a disease associated with decrease in tear;
[18] a use of a compound selected from the group consisting of a phenylethanolaminotetralincarboxamide derivative as described in any of the above [4] to [6], [3-[(2R)-[[(2R)-(3-chlorophenyl)-2-hydroxyethyl]-amino]propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]propyl]-2,3-dihydro-1,4-benzodixine-2-(R)-carboxylic acid, and a pharmaceutically acceptable salt thereof for manufacturing of a pharmaceutical composition for the enhancement of protein in tear;
[19] a use of a compound selected from the group consisting of a phenylethanolaminotetralincarboxamide derivative as described in any of the above [1] to [3], [3-[(2R)-[[(2R)-(3-chlorophenyl)-2-hydroxyethyl]-amino]propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]propyl]-2,3-dihydro-1,4-benzodixine-2-(R)-carboxylic acid, and a pharmaceutically acceptable salt thereof for manufacturing of a pharmaceutical composition for the facilitation of mucin secretion in tear; and the like.

### Effect of the invention

A compound selected from the group consisting of the compounds represented by the above general formula (I), [3-[(2R)-[[(2R)-(3-chlorophenyl)-2-hydroxyethyl]amino]-propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]propyl]-2,3-dihydro-1,4-benzodixine-2-(R)-carboxylic acid, and a pharmaceutically acceptable salt thereof (hereinafter referred also to as "the above active ingredient") exerts effects facilitating secretion of tear and protein in tear and is useful for the prevention or treatment of diseases associated with decrease in tear such as dry eye or the like. In addition, since the above active ingredients extremely increase the quantity of protein in tear, especially mucin secretion, more than terbutaline, a selective β₂ AR stimulant having a similar level of β₂ AR stimulating activity, they are useful as a pharmaceutical composition for increasing the quantity of protein in tear, or for facilitating mucin secretion in tear.

### Best mode to operate the invention

The above active ingredients exert remarkable facilitating effects of tear and protein secretion in tear. As preferable compounds in the above active ingredients, 2-[(2S)-2-[[(2R)-2-hydroxy-2-(4-hydroxyphenyl)ethyl]-amino]-1,2,3,4-tetrahydronaphthalen-7-yloxy]-N,N-dimethylacetamide (hereinafter referred to as "Compound 1"), 1-[2-[(2S)-2-[[(2R)-2-hydroxy-2-(4-hydroxyphenyl)ethyl]amino]-1,2,3,4-tetrahydronaphthalen-7-yloxy]acetyl]piperidine and 4-[2-[(2S)-2-[[(2R)-2-hydroxy-2-(4-hydroxyphenyl)ethyl]-amino]-1,2,3,4-tetrahydronaphthalen-7-yloxy]acetyl]-morpholine, and a pharmaceutically acceptable salt thereof; AJ-9677, FR-149175, N-5984 and the like can be illustrated, and they exert superior effects than existing selective β₂ AR stimulants. The compounds represented by the above general formula (I) of the present invention can be prepared according to a known method (see Patent reference 1) or a similar method thereto. In addition, AJ-9677, FR-149175 and N-5984 can be also prepared according to a known method or a similar method thereto.

A dosage of any of the above active ingredients may be determined as needed according to the active ingredient, and body weight, age, sex and degree of diseases of each patient. For example, the range of dosage in adults is preferable 1 to 1000 mg/day in oral administration and 0.001 to 1% in ocular administration.

In the compounds represented by the above general formula (I), the term "di(lower alkyl)amino group" means an amino group disubstituted by straight or branched alkyl having 1 to 6 carbons, and for example, a dimethylamino group, a diethylamino group, an ethylmethylamino group and the like can be illustrated. The term "lower alkylene group" means a straight or branched alkylene group having 1 to 6 carbons, and for example, a methylene group, an ethylene group, a triethylene group and the like can be illustrated. The term "3 to 7-membered alicyclic amino group which may have an oxygen atom in the ring" means a cyclic alkylamino group having 2 to 6 carbons, and for example, a 1-pyrrolidinyl group, a piperidino group, a morpholino group and the like can be illustrated.

A pharmaceutical composition of the present invention exerts a facilitating activity of tear secretion and protein secretion in tear, and thus, is useful for the prevention or treatment of a disease associated with decrease in tear. In the present invention, the term "disease associated with decrease in tear" means ophthalmic dry symptoms caused qualitative and/or quantitative abnormality and a disorder of the keratoconjunctival epithelium associated therewith and also includes one caused by any causes of decrease in tear secretion and enhanced evaporation or excretion of tear, and, for example, dry eye, dry disorders of cornea and conjunctiva, disorders of the keratoconjunctival epithelium, syndrome with decrease in tear secretion, xerophthalmia, dry eye due to aging, ophthalmopathy in Stevens-Johnson syndrome, ophthalmopathy in Sjögren's syndrome, keratoconjunctival ulcer, oligodacrya, keratoconjunctivitis sicca, ocular pemphigus, blepharitis marginalis, insufficient occlusion of eye lids, sensory neuroparalysis, allergic conjunctivitis, dryness post-viral conjunctivitis, post-cataract surgery, in wearing of contact lens or in operation of visual display terminal (VDT) and the like can be illustrated. Dry eye includes dry eye based on the diagnostic criteria as described in Non-patent reference 1 as well as dry eye diagnosed or suspected based on characteristics such as qualitative or quantitative abnormality (decrease) or disorders of the keratoconjunctival epithelium associated therewith.

The above active ingredients can be converted into a pharmaceutically acceptable salts thereof in the usual ways. As such a salt, acid additive salts with mineral acids such as hydrochloric acid, hydrobromic acid, hydroiodic acid, sulfuric acid, nitric acid, phosphoric acid and the like; acid additive salts with organic acids such as formic acid, acetic acid, methanesulfonic acid, p-toluenesulfonic acid, propionic acid, citric acid, succinic acid, tartaric acid, fumaric acid, butyric acid, oxalic acid, malonic acid, maleic acid, lactic acid, malic acid, carbonic acid, glutamic acid, aspartic acid and the like; and salts with inorganic bases such as sodium, potassium and the like can be illustrated.

When the above active ingredients are employed in the practical treatment, as appropriate dosage forms, for example, oral formulations such as powders, granules, fine granules, dry syrups, tablets, capsules; parenteral formulations such as eye drops, injections, poultices, suppositories and the like are illustrated, and oral formulations or eye drops are preferable. Particularly, oral formulations are preferable for a patient sensitive to mucosal irritative effects caused by antiseptics or the like. In addition, a formulation can be used by preparing various dosage forms in the usual way optionally by admixing or by diluting and dissolving the above active ingredient with formulation carriers including necessary excipients, disintegrators, binders, lubricants, diluents, buffers, isotonic agents, antiseptics, humectants, emulsifiers, dispersing agents, stabilizers and solubilizers or the like.

### Examples

The present invention is further illustrated in more detail by way of the following Examples. However, the present invention is not limited thereto.

### [Example 1] Measurement of tear, and protein and mucin in tear in rabbits

Three male Japanese white rabbits (about 3 kg) were allocated to each group. Each 50 µL of 1/2 sulfate salt of Compound 1 (0.1% phosphate buffer solution, pH7.4), terbutaline sulfate (0.1% phosphate buffer solution, pH7.4) or a solvent thereof (a phosphate buffer, pH7.4) was administered to both eyes of the rabbit which was anesthetized with pentobarbital (40 mg/kg, i.v.). Two pieces each of pre-weighed filter paper (Wattman No. 41, 0.22 µm thick, 2.5 x 15 mm) were inserted to the lower conjunctival sac of the right or left eye, and the difference in weight of the filter papers before and after insertion (post-insertion weight - pre-insertion weight) was defined as the quantity of tear secretion. The quantity of tear secretion was measured for 5 min before drug administration and for 5 min at 5 min after drug administration. Fifty (50) µL of a local anesthetic agent, 0.4% oxybuprocaine hydrochloride (Santen Co.), was instilled into eyes 5 min before each measurement. Tear and the instilled local anesthetic were wiped immediately before the filter paper was inserted.

Filter papers recovered after each measurement were placed into tubes. A phosphate buffer (pH 7.4) (500 µL) was added to each of the tubes, and they were mixed by vortex mixer for 30 sec. After the filter papers were removed and the mixture was centrifuged at 470 x g for 5 min, protein concentrations in supernatant were measured using Micro BCA Protein Assay Reagent Kit (Pierce Co.). The quantity of protein in tear was calculated based on the measured protein concentration and quantity of tear secretion. The difference in the quantity of tear secretion before administration of a solvent, 1/2 sulfate of Compound 1 or terbutaline sulfate and that after administration was defined as a change in the quantity of tear secretion or protein in tear, respectively. Each milligram of the change in the quantity of tear was taken as 1 µL.

In addition, at the same timing of measurement of the quantity of tear secretion, 3 mm diameter cellulose acetate filter paper was pressed on the conjunctiva for 30 sec. by impression cytology method, and periodic acid/Schiff (PAS) staining was performed with the recovered filter papers. After filter papers were cleared and embedded, the number of PAS positive goblet cells existing in a certain visual field of a microscope was counted.

The count of PAS positive goblet cells (%) shows a percentage (%) of that in the solvent intraocularly administered group. Decreases in the count of PAS positive goblet cells indicate that mucin secretion from the conjunctiva is facilitated. The results are shown in Table 1. Each value is mean of 4 animals, and 1/2 sulfate of Compound 1 increased extremely about 30% more in the quantity of tear secretion, about 200% more the quantity of protein in tear and about 50% more mucin secretion from conjunctiva in comparison with the solvent and terbutaline sulfate.

**[Table 1]**

| Name of compound | Concentration of compound (w/v%) | Tear secretion (µL) | Change in protein in tear (µg) | Count of PAS-positive goblet cells (%) |
|---|---|---|---|---|
| Solvent | - | 0.17 | 0.28 | 100.0 |
| 1/2 sulfate of Compound 1 | 0.1 | 4.87 | 2.57 | 64.8 |
| Terbutaline sulfate | 0.1 | 3.80 | 1.04 | 77.3 |

### [Example 2] Measurement of tear, and protein and mucin in tear in rabbits

By the same method as described in Example 1, N-5984 was evaluated, and the results are shown in Table 2. N-5984 extremely increased the quantities of tear secretion, protein in tear and mucin secretion from conjunctiva.

**[Table 2]**

| Name of compound | Concentration of compound (w/v%) | Tear secretion (µL) | Change in protein in tear (µg) | Count of PAS-positive goblet cells (%) |
|---|---|---|---|---|
| Solvent | - | -0.17 | -0.01 | 100.0 |
| N-5984 | 0.1 | 4.42 | 2.72 | 57.1 |

### Industrial applicability

The pharmaceutical compositions of the present invention are extremely useful as agents for the prevention or treatment of diseases associated with decrease in tear.

## Claims

1. A pharmaceutical composition for the prevention or treatment of a disease associated with decrease in tear, which comprises as an active ingredient a compound selected from a group consisting of a phenylethanolaminotetralincarboxamide derivative represented by the general formula: 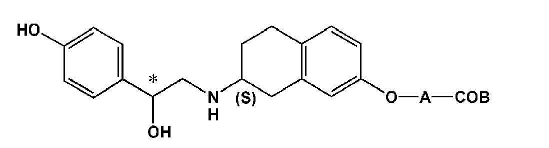 wherein A represents a lower alkylene group, B represents an amino group, a di(lower alkyl)amino group or a 3 to 7-membered alicyclic amino group which may have an oxygen atom in the ring, a carbon atom with the mark "*" represents a carbon atom of S-configuration or R-configuration, or a mixture thereof, and a carbon atom with (S) represents a carbon atom of S-configuration, [3-[(2R)-[[(2R)-(3-chlorophenyl)-2-hydroxyethyl]amino]propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]propyl]-2,3-dihydro-1,4-benzodixine-2-(R)-carboxylic acid, and a pharmaceutically acceptable salt thereof.

2. A pharmaceutical composition as claimed in claim 1 wherein the active ingredient is a phenylethanolaminotetralincarboxamide derivative represented by the general formula: 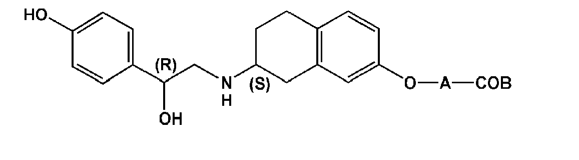 wherein A represents a lower alkylene group, B represents an amino group, a di(lower alkyl)amino group or a 3 to 7-membered alicyclic amino group which may have an oxygen atom in the ring, a carbon atom with (R) represents a carbon atom of R-configuration and a carbon atom with (S) represents a carbon of S-configuration, or a pharmaceutically acceptable salt thereof.

3. A pharmaceutical composition as claimed in claim 2 wherein the active ingredient is a compound represented by one of the formulas: 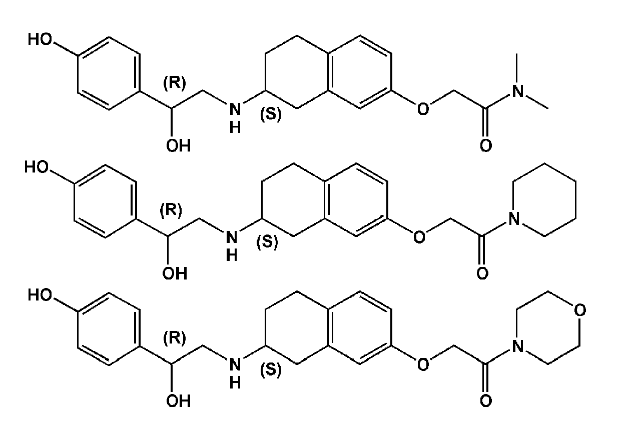 or a pharmaceutically acceptable salt thereof.

4. A pharmaceutical composition for the enhancement of protein in tear, which comprises as an active ingredient a compound selected from a group consisting of a phenylethanolaminotetralincarboxamide derivative represented by the general formula: 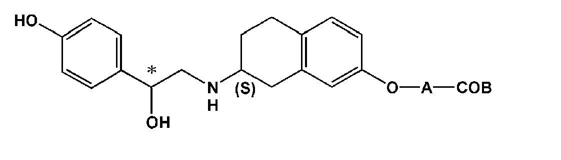 wherein A represents a lower alkylene group, B represents an amino group, a di(lower alkyl)amino group or a 3 to 7-membered alicyclic amino group which may have an oxygen atom in the ring, a carbon atom with the mark "*" represents a carbon atom of S-configuration or R-configuration, or a mixture thereof, and a carbon atom with (S) represents a carbon atom of S-configuration, [3-[(2R)-[[(2R)-(3-chlorophenyl)-2-hydroxyethyl]amino]propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]propyl]-2,3-dihydro-1,4-benzodixine-2-(R)-carboxylic acid, and a pharmaceutically acceptable salt thereof.

5. A pharmaceutical composition as claimed in claim 4 wherein the active ingredient is a phenylethanolaminotetralincarboxamide derivative represented by the general formula: 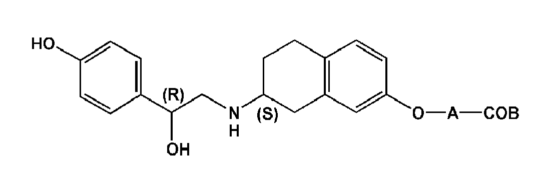 wherein A represents a lower alkylene group, B represents an amino group, a di(lower alkyl)amino group or a 3 to 7-membered alicyclic amino group which may have an oxygen atom in the ring, a carbon atom with (R) represents a carbon atom of R-configuration and a carbon atom with (S) represents a carbon of S-configuration, or a pharmaceutically acceptable salt thereof.

6. A pharmaceutical composition as claimed in claim 5 wherein the active ingredient is a compound represented by one of the formulas: 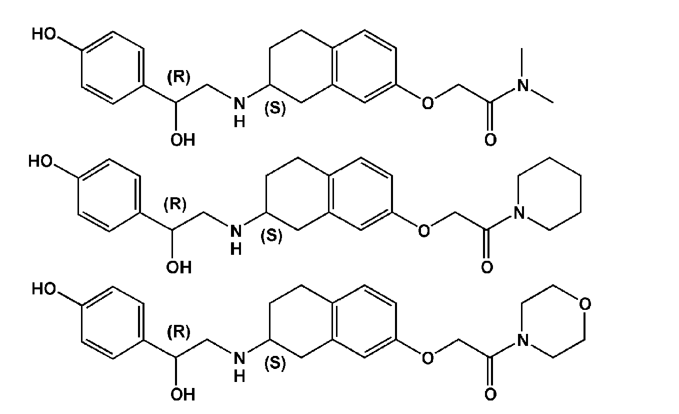 or a pharmaceutically acceptable salt thereof.

7. A pharmaceutical composition for the facilitation of mucin secretion in tear, which comprises as an active ingredient a compound selected from a group consisting of a phenylethanolaminotetralincarboxamide derivative represented by the general formula: 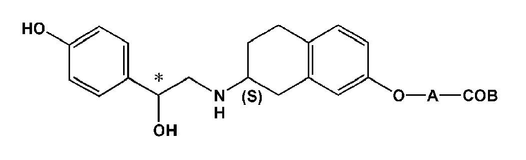 wherein A represents a lower alkylene group, B represents an amino group, a di(lower alkyl)amino group or a 3 to 7-membered alicyclic amino group which may have an oxygen atom in the ring, a carbon atom with the mark "*" represents a carbon atom of S-configuration or R-configuration, or a mixture thereof, and a carbon atom with (S) represents a carbon atom of S-configuration, [3-[(2R)-[[(2R)-(3-chlorophenyl)-2-hydroxyethyl]amino]propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]propyl]-2,3-dihydro-1,4-benzodixine-2-(R)-carboxylic acid, and a pharmaceutically acceptable salt thereof.

8. A pharmaceutical composition as claimed in claim 7 wherein the active ingredient is a phenylethanolaminotetralincarboxamide derivative represented by the general formula: 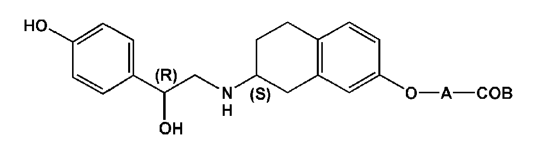 wherein A represents a lower alkylene group, B represents an amino group, a di(lower alkyl)amino group or a 3 to 7-membered alicyclic amino group which may have an oxygen atom in the ring, a carbon atom with (R) represents a carbon atom of R-configuration and a carbon atom with (S) represents a carbon of S-configuration, or a pharmaceutically acceptable salt thereof.

9. A pharmaceutical composition as claimed in claim 8 wherein the active ingredient is a compound represented by one of the formulas: 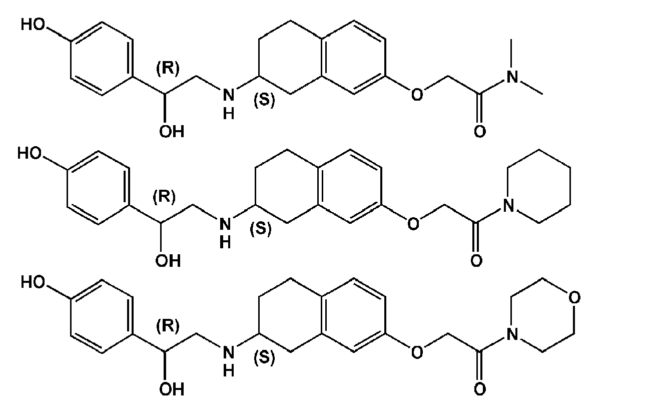 or a pharmaceutically acceptable salt thereof.

10. A pharmaceutical composition as claimed in any of claims 1 to 3 wherein the disease associated with decrease in tear are one or more diseases selected from the group consisting of dry eye, dry disorders of cornea and conjunctiva, disorders of the keratoconjunctival epithelium, syndrome with decrease in tear secretion, xerophthalmia, dry eye due to aging, ophthalmopathy in Stevens-Johnson syndrome, ophthalmopathy in Sjögren's syndrome, keratoconjunctival ulcer, oligodacrya, keratoconjunctivitis sicca, ocular pemphigus, blepharitis marginalis, insufficient occlusion of eye lids, sensory neuroparalysis, allergic conjunctivitis, and dryness post-viral conjunctivitis, post-cataract surgery, in wearing of contact lens or in operation of visual display terminal (VDT).

11. A pharmaceutical composition as claimed in any of claims 1 to 10 wherein the dosage form is an oral formulation.

12. A pharmaceutical composition as claimed in any of claims 1 to 10 wherein the dosage form is a parenteral formulation.

13. A pharmaceutical composition as claimed in claim 12 wherein the parenteral formulation is an eye drop.

14. A method for the prevention or treatment of a disease associated with decrease in tear, which comprises administering an effective amount of a compound selected from the group consisting of a phenylethanolaminotetralincarboxamide derivative as claimed in any of claims 1 to 3, [3-[(2R)-[[(2R)-(3-chlorophenyl)-2-hydroxyethyl]amino]-propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]propyl]-2,3-dihydro-1,4-benzodixine-2-(R)-carboxylic acid, and a pharmaceutically acceptable salt thereof.

15. A method for the enhancement of protein in tear, which comprises administering an effective amount of a compound selected from the group consisting of a phenylethanolaminotetralincarboxamide derivative as claimed in any of claims 4 to 6, [3-[(2R)-[[(2R)-(3-chlorophenyl)-2-hydroxyethyl]amino]propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]propyl]-2,3-dihydro-1,4-benzodixine-2-(R)-carboxylic acid, and a pharmaceutically acceptable salt thereof.

16. A method for the facilitation of mucin secretion in tear, which comprises administering an effective amount of a compound selected from the group consisting of a phenylethanolaminotetralincarboxamide derivative as claimed in any of claims 7 to 9, [3-[(2R)-[[(2R)-(3-chlorophenyl)-2-hydroxyethyl]amino]propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]propyl]-2,3-dihydro-1,4-benzodixine-2-(R)-carboxylic acid, and a pharmaceutically acceptable salt thereof.

17. A use of a compound selected from the group consisting of a phenylethanolaminotetralincarboxamide derivative as claimed in any of claims 1 to 3, [3-[(2R)-[[(2R)-(3-chlorophenyl)-2-hydroxyethyl]amino]propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]propyl]-2,3-dihydro-1,4-benzodixine-2-(R)-carboxylic acid, and a pharmaceutically acceptable salt thereof for manufacturing of a pharmaceutical composition for the prevention or treatment of a disease associated with decrease in tear.

18. A use of a compound selected from the group consisting of a phenylethanolaminotetralincarboxamide derivative as claimed in any of claims 4 to 6, [3-[(2R)-[[(2R)-(3-chlorophenyl)-2-hydroxyethyl]amino]propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]propyl]-2,3-dihydro-1,4-benzodixine-2-(R)-carboxylic acid, and a pharmaceutically acceptable salt thereof for manufacturing of a pharmaceutical composition for the enhancement of protein in tear.

19. A use of a compound selected from the group consisting of a phenylethanolaminotetralincarboxamide derivative as claimed in any of claims 1 to 3, [3-[(2R)-[[(2R)-(3-chlorophenyl)-2-hydroxyethyl]amino]propyl-1H-indol-7-yloxy]acetic acid, ethyl [(S)-8-[(R)-2-(3-chlorophenyl)-2-hydroxyethylamino]-6,7,8,9-tetrahydro-5H-benzocyclohepten-2-yloxy]acetate and 6-[2-(R)-[[2-(R)-(3-chlorophenyl)-2-hydroxyethyl]amino]propyl]-2,3-dihydro-1,4-benzodixine-2-(R)-carboxylic acid, and a pharmaceutically acceptable salt thereof for manufacturing of a pharmaceutical composition for the facilitation of mucin secretion in tear.
